# EUROPEAN PATENT SPECIFICATION

(11) **EP 1 607 037 B1**
(45) Date of publication and mention of the grant of the patent: **11.02.2015**
(21) Application number: 05010517.0
(22) Date of filing: 13.05.2005
(51) Int. Cl.: A61B 1/018, A61B 1/00, G02B 23/24

(54) **Endoscope apparatus**
Endoskop
Endoscope

(30) Priority: 26.05.2004 JP 2004156151
(43) Date of publication of application: 21.12.2005
(73) Proprietor: Olympus Corporation, Shibuya-ku, Tokyo (JP)
(72) Inventor: Okada, Yuta, Hachioji-shi, Tokyo 192-8512 (JP); Sekine, Ryuta, Hachioji-shi, Tokyo 192-8512 (JP); Matsui, Raifu, Hachioji-shi Tokyo 192-8512 (JP)
(74) Representative: von Hellfeld, Axel

(56) References cited:
- US-A- 4 425 025
- US-A- 6 117 071
- US-A1- 2002 123 664
- US-A1- 2004 097 790
- US-B1- 6 352 503

## Description

### CROSS-REFERENCE TO RELATED APPLICATION

This application is based upon and claims the benefit of priority from prior Japanese Patent Application No.2004-156151, filed on May 26, 2004.

### BACKGROUND OF THE INVENTION

### 1. Field of the Invention

The present invention relates to an endoscope apparatus using a therapeutic tool therewith. For example, the therapeutic tool is an instrument introduced by an insertion guide tube with a bending function.

### 2. Description of the Related Art

In the related art, an endoscope treatment apparatus to be used with an insertion guide tube having a bending function for guiding an instrument is known (see JP-A-2000-33071). In this endoscope treatment apparatus, an operator adjusts the direction or the position of the distal end of the instrument guided into a body cavity by bending the insertion guide tube as needed.

As described above, when the operator operates a therapeutic tool, which is the instrument guided by the insertion guide tube having a bending function in the body cavity, the treatment portion of the therapeutic tool must always be in the observable field of view of the endoscope.

However, since the observable area of the endoscope in the body cavity is limited to a narrow area near the distal end thereof, when the distal treatment portion of the therapeutic tool is moved away from the distal end of the endoscope even by a small extent, the treatment portion of the therapeutic tool can be seen only as a small image.

In order to enable observation of the state of the tool in a viewer-friendly scale, the operator is required to perform operational work by bending the insertion guide tube and placing the treatment portion of the therapeutic tool as close as possible to the distal end of the endoscope.

Therefore, the operator must bend a bending portion of the insertion guide tube strongly and sharply in a small range and in a small radius. Consequently, an excessive force is apt to be exerted to parts of the bending portion or an operation wire. In addition, the instrument cannot be moved back and forth smoothly at the sharply bent portion. As another problem, there is a limit in design or manufacture of the bending portion which can be bent sharply. From the reasons described above, when attempt is made by the operator to operate the instrument without problem by the insertion guide tube, the instrument must be operated at an area apart from the distal end of the endoscope.

After all, in the existing endoscope, it is difficult to achieve both of observing the state of the instrument at a viewer-friendly scale and performing the treatment operation without problems.

Such circumstances are part of the reasons why operations with existing endoscopes is more difficult than normal abdominal surgical operations.

US 2002/123664, from which the preamble of claim 1 is known, discloses an endoscope having an objective lens system with an objective lens drive mechanism for moving a lens of the objective lens system in order to vary the focal depth, the image magnification, or the view field angle of the objective lens system, and a biopsy port located on a distal surface of the endoscope together with an illumination window and an optical window of the objective lens system.

US 6,352,503 discloses a endoscopic surgery apparatus for performing a treating operation in a body cavity with the use of treating tools in combination with an endoscope, in which insertion sections allowing the insertion of treating tools are externally arranged from the endoscope in a side-by-side fashion with an insertion section of the endoscope set between these insertion sections and a balloon provided near a distal end of the insertion section of the endoscope to adjust a relative distance between the insertion section relative to the insertion section of the endoscope. An outer tube unit serves to hold the insertion sections of the treatment tools and the insertion section of the endoscope arranged in the side-by-side arrangement. The outer tube unit is adapted to be fixed to the insertion section of the endoscope proximally to a bending section of the endoscope.

### BRIEF SUMMARY OF THE INVENTION

An endoscope apparatus according to claim 1 is provided. Preferred embodiments are set forth in the dependent claims.

The endoscope apparatus of the invention includes an endoscope including an observation optical system in which the angle of observable field of view for observing a therapeutic tool can be changed and an operating mechanism for changing the field angle of the observation optical system, and an insertion guide tube for guiding the therapeutic tool into a body cavity, wherein the insertion guide tube is detachably positioned and supported in a predetermined position relative to the endoscope.

Further, in the endoscope apparatus , when changing the angle of field of view to observe in an enlarged scale, the observation optical system can be located in a same focal depth range before and after enlargement.

In this arrangement, since the focal point is not displaced even in the case of observing in an enlarged scale, the object is prevented from becoming blurred.

Accordingly, the tool to be operated by the insertion guide tube can be viewed at an optimum scale whether the area to be observed is close or far.

According to an embodiment of the endoscope apparatus, in the endoscope apparatus according to the first aspect described above, the insertion guide tube has a bending function positioned and supported with an insertion portion of the endoscope.

In this arrangement, since display on a screen of an easy-to-observe size is enabled without bending the insertion guide tube having a bending function strongly and sharply in a small range and in a small radius, the operator can move the therapeutic tools back and forth smoothly.

According to a further embodiment of the endoscope apparatus, in the endoscope apparatus described above, the predetermined position can comprise an endoscope position located distally to a bending portion of the endoscope corresponding to an insertion guide tube position located proximally to a bendable area of the insertion guide tube.

In this arrangement, stable and smooth movement of the bendable area is achieved.

According to a further embodiment of the endoscope apparatus, in the endoscope apparatus described above, an image pickup device for picking up an image of the observable field of view into the endoscope apparatus is further provided, and an angle of field of view corresponding to a length of the longest diagonal line in an image pickup area of the image pickup device is set to a range between 60° and 100° for a narrow angle mode.

This arrangement is practical and accordingly, the operator can easily use the endoscope apparatus.

According to a further embodiment of the endoscope apparatus, in the endoscope apparatus described above, an image pickup device for picking up an image of the observable field of view into the endoscope apparatus is further provided, and an angle of field of view corresponding to a length of a longest diagonal line in an image pickup area of the image pickup device is set to a range between 100° and 150° for a wide angle mode, and between 60° and 100° for a narrow angle mode.

In this arrangement, an optimal observation state can be obtained in the wide angle mode and the narrow angle mode, respectively.

According to a further embodiment of the endoscope apparatus, in the endoscope apparatus described above, the focal depth of the observation optical system includes a position 40 mm distally from a position of a distal end of a lens at a distal-most position of the observation optical system.

In this arrangement, since the position of the objective portion is located 40 mm distally from the position of the distal end of the lens, the actual use of the endoscope is convenient.

### BRIEF DESCRIPTION OF THE SEVERAL VIEWS OF THE DRAWINGS

These and other features, aspects, and advantages of the apparatus and methods of the present invention will become better understood with regard to the following description, appended claims, and accompanying drawings where:
Fig. 1 is a partial perspective view showing a distal end of an endoscope treatment apparatus according to a first embodiment;
Fig. 2 is a perspective view of the distal end of the endoscope treatment apparatus in a disassembled state according to the first embodiment;
Fig. 3 is a cross sectional view of an objective unit integrated in the distal portion of an endoscope vertical to the axis thereof according to the first embodiment;
Fig. 4A is an explanatory drawing of the endoscope according to the first embodiment in which an angle of an objective observation optical system is large;
Fig. 4B is an explanatory drawing of the endoscope treatment apparatus according to the first embodiment, showing a display being made on a display unit thereof;
Fig. 5A is an explanatory drawing of the endoscope according to the first embodiment of the invention in which the angle of the objective observation optical system is small;
Fig. 5B is an explanatory drawing of the endoscope treatment apparatus according to the first embodiment, showing a display being made on the display unit;
Fig. 6 is an explanatory drawing showing a relation between an angle W of the observable field of view of the objective observation optical system and a bending portion of an insertion guide tube in the endoscope according to the first embodiment;
Fig. 7A is an explanatory drawing showing a state of the bending portion of the insertion guide tube displayed on the screen of the display unit corresponding to one of the angle W of the observable field of view of the objective observation optical system in the endoscope according to the first embodiment;
Fig. 7B is an explanatory drawing showing a state of the bending portion of the insertion guide tube displayed on the screen of the display unit corresponding to one of the angle W of the observable field of view of the objective observation optical system in the endoscope according to the first embodiment;
Fig. 7C is an explanatory drawing showing a state of the bending portion of the insertion guide tube displayed on the screen of the display unit corresponding to one of the angle W of the observable field of view of the objective observation optical system in the endoscope according to the first embodiment;
Fig. 8 is a partial perspective view showing a distal end of an endoscope treatment apparatus of another type;
Fig. 9 is a partial perspective view showing a distal end of an endoscope treatment apparatus of still another type;
Fig. 10 is a partial perspective view showing a distal portion of an endoscope treatment apparatus according to a second embodiment in a state of being used; and
Fig. 11 is a partial perspective view showing a distal portion of the endoscope treatment apparatus according to a third embodiment in a state of being used.

### DETAILED DESCRIPTION OF THE INVENTION

Preferred embodiments will be described below with reference to the accompanying drawings.

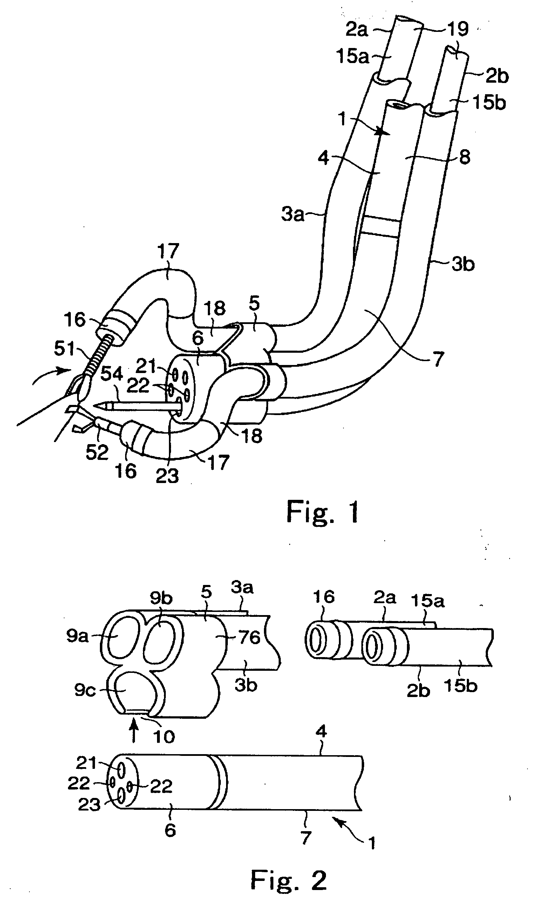

Referring now to Fig. 1 to Fig. 9, an endoscope apparatus according to a first embodiment will be described.

As shown in Fig. 1, the endoscope apparatus according to this embodiment includes a flexible endoscope 1, two insertion guide tubes 2a, 2b, and two flexible guide tubes 3a, 3b through which the insertion guide tubes 2a, 2b are inserted independently. The flexible guide tubes 3a, 3b through which the insertion guide tubes 2a, 2b are inserted independently and an insertion portion 4 of the endoscope 1 are detachably connected by a connecting member 5 as an auxiliary member for positioning and supporting the same.

The endoscope 1 includes the elongated insertion portion 4, and a control section (not shown) provided at an operator's side of (proximally to) the insertion portion 4. The insertion portion 4 of the endoscope 1 includes, as shown in Fig. 1, a distal portion 6 positioned at a distal end thereof, a bending portion 7 located on the operator's side of the distal portion 6, and a flexible portion 8 located further on the operator's side of the bending portion 7. The bending portion 7 of the endoscope 1 is, like the general endoscope, bent by an operation at the control section.

The connecting member 5 includes, as shown in Fig. 2, two openings 9a, 9b fixedly connecting respective extremities of the guide tubes 3a, 3b, and an opening 9c for fitting the distal portion 6 of the insertion portion 4 of the endoscope 1 for fixedly engaging the same. The respective openings 9a, 9b, 9c are parallel to each other, and the centers of the respective openings 9a, 9b, 9c are disposed so as to position at apexes of a triangle. A sidewall portion of the opening 9c to which the distal portion 6 of the endoscope 1 is fitted is formed with a slit 10 opening toward the side. The slit 10 is intended for facilitating widening of the opening 9c through a plastic deformation when fitting or removing the distal portion 6 of the endoscope 1 to/from the opening 9c. Although the connecting member 5 is normally detachably attached to the distal portion 6 of the endoscope 1, averting the bending portion 7 or the flexible portion 8, it is also possible to engage the same to the end of the bending portion 7 on the operator's side end or to the flexible portion 8.

On the other hand, as shown in Fig. 1, insertion portions 15a, 15b of the respective insertion guide tubes 2a, 2b each include a distal portion 16 positioned at a distal end thereof, first bending portion 17 as bendable area positioned on the operator's side of the distal portion 16, second bending portion 18 located on the operator's side of the first bending portion 17, and flexible portion 19 provided continuously from the second bending portions 18 toward on the operator's side. The insertion portions 15a, 15b of the insertion guide tubes 2a, 2b are inserted separately through the respective guide tubes 3a, 3b. At least the portions from the distal portions 16 to the first bending portion 17 and the second bending portion 18 can project from the distal opening ends of the guide tubes 3a, 3b. The insertion guide tubes 2a, 2b are adapted to be used as manipulators for operating the therapeutic tool.

The first bending portion 17 and the second bending portion 18 of the respective insertion guide tubes 2a, 2b are tubular portions that can be bent freely in the vertical and lateral directions as is known in the art. They can be adapted to be bendable by utilizing, for example, a plurality of bending pieces (not shown). The first bending portions 17 and the second bending portions 18 are respectively bent independently by pushing and pulling operation wires (not shown) respectively by remotely controlling the respective operating units of the insertion guide tubes 2a, 2b on the operator's side.

The endoscope 1 in this embodiment is a straight-view type, and is provided with an observation window 21, an illumination window 22, and an instrument channel port 23 on the distal end surface of the distal portion 6, as shown in Fig. 1 and Fig. 2 as in the case of a general endoscope.

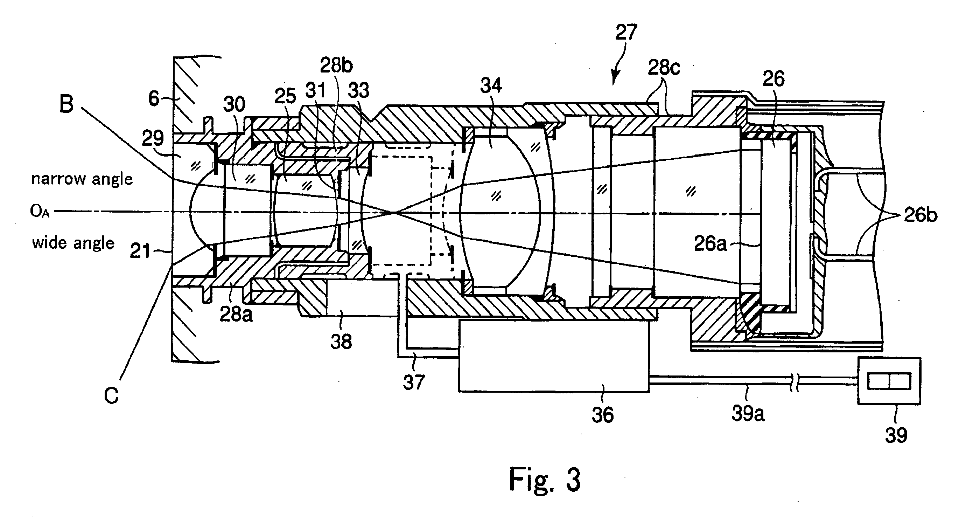

As shown in Fig. 3, the distal portion 6 is provided with an objective unit 27 including an objective observation optical system 25 for picking up (e.g., capturing) an image in the observable field of view from the observation window 21 and an image pickup device 26 for picking up the image in the observable field of view picked up by the objective observation optical system 25 integrated therein. The objective observation optical system 25 is of a scope system with magnification function, in which lenses are assembled to lens retaining frames 28a, 28b, 28c divided into a plurality of pieces.

An objective lens group 30 including a transparent window member 29 constituting the observation window 21 and a field stop 31 are fixedly attached to the first lens retaining frame 28a. The second lens retaining frame 28b is movable in the direction of the optical axis O_{A}, and a movable lens or a movable lens group 33 is attached to the second lens retaining frame 28b. The image pickup device 26 and an image forming lens group 34 for forming a field image in an image pickup area 26a of the image pickup device 26 are fixedly attached to the third lens retaining frame 28c.

As shown in Fig. 3, the movable second lens retaining frame 28b is slidably fitted to the inner surface of the third lens retaining frame 28c, and is assembled so as to be movable in the direction of the optical axis O_{A} of the objective observation optical system 25. The second lens retaining frame 28b is moved by an operating mechanism described later. The angle of field of view to be observed is changed by a movement of the movable lens 33 in the direction of the optical axis O_{A}. The movable lens 33 includes a single concave lens disposed between the objective lens group 30 as a first lens group and the image forming lens group 34. The movable lens 33 constitutes a field angle changing mechanism, so that the angle of observable field of view taken into the endoscope 1 is changed by sliding the movable lens 33 in the direction of the optical axis O_{A}. The field angle changing mechanism is adapted to be capable of changing the field angle continuously or stepwise (incrementally).

The operating mechanism for operating the field angle changing mechanism is an electric actuator 36 for moving the second lens retaining frame 28b, as shown in Fig. 3. The electric actuator 36 includes an operating arm 37 that moves back and forth in the direction of the optical axis. The operating arm 37 extends into the lens retaining frame 28C through a slit 38 formed on the wall of the fixed third lens retaining frame 28c, and is connected to the movable lens retaining frame 28b. The operator moves the movable lens 33 in the direction of the optical axis by operating selectively the operating arm 37 in the fore and aft optical direction by the electric actuator 36, so that the angle of observable field of view by the objective observation optical system 25 can be changed to a desired value. As shown in Fig. 3, the electric actuator 36 is selectively driven by the operator operating a control section 39 such as a switch or the like provided on a grasping portion provided on the operator's side of the endoscope 1 corresponding thereto. The electric actuator 36 is connected to the control section 39 via a drive control line 39a.

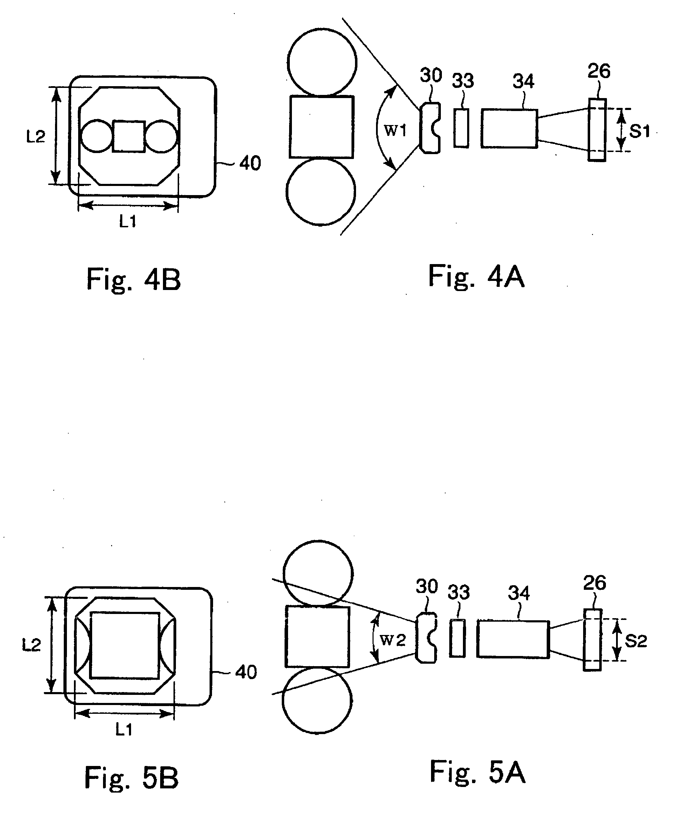

An image of the observable field of view taken from the observation window 21 by the objective observation optical system 25 is formed in the image pickup area 26a of the image pickup device 26, transmitted to a signal processing circuit (not shown) outside the endoscope through a transmission line 26b, and converted into a screen image signal. The screen image is displayed on a screen 40 of the display unit shown in Fig. 4B, and Fig. 5B respectively.

Subsequently, the field angle selected by the field angle changing mechanism of the objective observation optical system 25 will be described. Normally, the angle referred to as the field angle when picking up the image of the observable field of view by the endoscope is an angle of range of the field of view which can be observed, corresponding to "W1" in the normal state (distant view) shown in Fig. 4A or "W2" in an enlarged scale shown in Fig. 5A. In other words, the field angle in this embodiment represents a range of light entering from the observable field of view in front (distally) of the observation window 21 through the objective lens group 30 as the first lens group, passing as is through the movable lens 33 and the respective lenses in the image forming lens group 34, and entering an effective image pickup area of the image pickup device 26 at the rearmost end to form an image on the light receiving surface, and is represented by the incident angle "W" with respect to the objective lens group 30. Specific consideration will be made by applying this relation to the objective observation optical system 25 shown in Fig.3. Light in the range wider than the "W1" in the normal state (distant view) actually comes into the objective lens group 30. The light is refracted according to the concave and convex surfaces of the respective lenses 30, 33, 34 in the optical system located along the optical axis O_{A}, while part of the light being cut by the field stop 31 or the like provided as needed at the respective portions, is proceeded to the light receiving surface of the image pickup device 26, and reaches the light receiving surface of the image pickup device 26, where an image is picked up.

However, since part of the light reaching the light receiving surface for picking up the image of the image pickup device 26, which reaches the ends of the light receiving surface, varies, the light reaching the ends of the light receiving surface is cut and a remaining part of the light is used for image pickup as a signal for the screen image. The area to which the light to be used for image pickup, that is, to be converted as the image pickup signal, is an effective image pickup area corresponding to the field angle "W" at that time. Referring also to Fig. 3, an optical path of the objective observation optical system 25 will be described. The movable lens 33 shown by a solid line in Fig. 3 is in a state of being moved to the front-most position, and the movable lens 33 shown by a broken line in Fig. 3 is in a state of being moved to the rearmost position. As shown by the solid line in Fig. 3, in the state in which the movable lens 33 is slid to the front-most position, the field angle is such that light comes into the first objective lens group 30 from the range of the widest angle. The light path B of this incident light, which comes from the range of the narrowest field angle, is shown on the upper half of the objective unit 27. In contrast, in a state in which the sliding portion of the movable lens 33 is slid to the rearmost position, light comes into the objective lens group 30 from the range of the widest field angle. The light path C of this incident light is shown on the lower half of the objective unit 27. As will be seen from the broadening of the field angle shown in Fig. 3, when the movable lens 33 is moved forward, the field angle is reduced, and light comes in only from the small angle of field of view. In contrast, when the movable lens 33 is moved rearward, the field angle is increased, whereby light comes in from the large angle of field of view.

Subsequently, in keeping with the fact described above, the relation between the width of the field angle "W" and enlargement of the image will be described referring to Fig. 4A and Fig. 4B.

When using the endoscope 1 in the state of a large field angle W1 shown in Fig. 4A, light in the range of large field angle "W1" can come into the objective unit 27. Therefore, the range which the observer can observe (the range displayed on the monitor screen 40 shown in Fig. 4B) corresponds to the entire range of the field angle "W1". In other words, a wide range of the subject is displayed on the monitor screen 40 as shown in Fig. 4B. However, since the effective image pickup range of the image pickup device 26 does not change and the sizes of display areas L1, L2 on the monitor screen 40 are constant, the subject to be observed is displayed in a small scale as a consequence.

Also, when the endoscope 1 is used in a state of the smaller field angle "W2" as shown in Fig. 5A, since the effective image pickup range of the image pickup device 26 does not change and the sizes of display areas L1, L2 on the monitor screen 40 are constant, the subject to be observed is consequently displayed in an enlarged scale as shown in Fig. 5B.

In other words, in order to observe in an enlarged scale, the field angle "W2" is adjusted to be smaller than the normal state by moving the movable lens 33. Then, since the area of the range of the narrow field angle "W2" is displayed on the monitor screen 40 as an image from the reasons described above, if the sizes of the display areas L1, L2 on the monitor screen 40 remain in the normal state, the object residing in the field of view is displayed in an enlarged scale, as shown in Fig. 5B.

As described above, the size of the object displayed on the monitor screen 40 can be changed by changing the field angle "W" under the conditions that the sizes of the display areas L1, L2 on the monitor screen 40 do not change, and that the range of the area used as the image signal on the image pickup surface of the image pickup device 26 is not changed ("S1" shown in Fig. 4A and "S2" shown in Fig. 5A are the same (S1=S2)).

Subsequently, the operation when using the endoscope treatment apparatus according to this embodiment will be described in detail.

The operator resiliently fits the distal portion 6 of the insertion portion 4 of the endoscope 1 into the opening 9c of the connecting member 5 shown in Fig. 2, and fixedly engages the same. In this case, the operator opens (plastically deforms) the slit 10 of the connecting member 5 and fits the distal portion 6 into the opening 9c. As shown in Fig. 1, the connecting member 5 is mounted to the distal portion 6 of the insertion portion 4 of the endoscope 1, and the two guide tubes 3a, 3b extend along the insertion portion 4 of the endoscope 1.

Then, the operator inserts the insertion portion 4 of the endoscope 1 and the guide tubes 3a, 3b together into the body cavity. In this case, since the connecting member 5 is mounted to the distal portion 6 of the insertion portion 4 of the endoscope 1, the connecting member 5 is in the state of being provided with the two guide tubes 3a, 3b, and hence is capable of easily leading and introducing the two guide tubes 3a, 3b. Since the distal ends of the flexible guide tubes 3a, 3b are located at the position of the distal portion 6 of the endoscope 1, the distal portions of the insertion guide tubes 2a, 2b or of the therapeutic tool which are introduced into the body cavity through the guide tubes 3a, 3b are introduced inevitably in front (distally) of the distal portion 6 of the endoscope 1 as described later. Such a configuration results in an enhancement of the introduction operability of the insertion guide tubes 2a, 2b and the tools including the endoscope 1.

When inserting the insertion guide tubes 2a, 2b and endoscope 1 into the body cavity, it is also possible to use an over-tube (not shown) for inserting the endoscope 1 and the guide tubes 3a, 3b in a state of fixing the guide tubes 3a, 3b to the insertion portion 4 of the endoscope 1 as described above.

Subsequently, after having introduced the insertion portion 4 of the endoscope 1 into the body cavity, as shown in Fig. 1, the operator inserts the insertion guide tubes 2a, 2b independently into the guide tubes 3a, 3b, and cause the entire area including the distal portions 16, the first bending portion 17, and the second bending portion 18 to project into the body cavity from the distal end of the guide tubes 3a, 3b.

Then, the operator introduces one or more operational tools into the body cavity through the insertion guide tubes 2a, 2b while observing the interior of the body cavity by the endoscope 1. Fig. 1 shows a state in which an operative tool, for example, grasping forceps 51, 52 as instruments are introduced through the guide tubes 3a, 3b independently. The operator can also cause an additional operational tool such as an electrosurgical knife 54 to project from the instrument channel port 23 of the endoscope 1.

In this case, since the movable portions of the insertion guide tubes 2a, 2b projecting from the distal ends of the guide tubes 3a, 3b are retained at the connecting member 5 as a fulcrum, stable and smooth movement of the movable portions of the insertion guide tubes 2a, 2b is achieved. In the case in which the grasping forceps 51, 52 as the instrument are operated in the reverse direction by the insertion guide tubes 2a, 2b, the power components exerted thereto in the opposite directions with the connecting member 5 as a common supporting point are cancelled with respect to each other. Therefore, the movable portions of the insertion guide tubes 2a, 2b move with the aforementioned fulcrum as a reference point, and hence a force exerted to the grasping forceps 51, 52 by the operator are reliably transmitted to an anatomy 53.

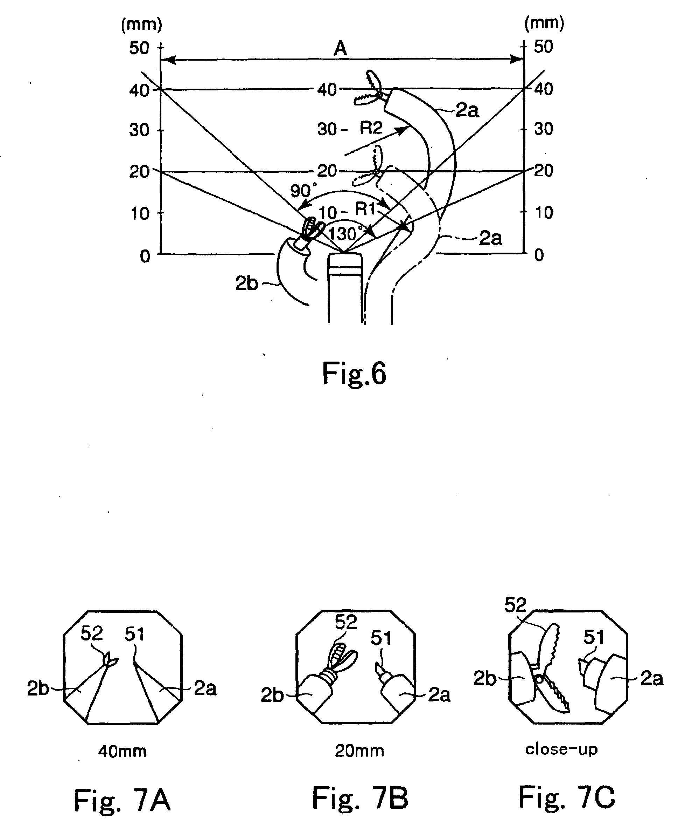

Subsequently, the state of observation in the body cavity by the endoscope 1 will be described. As shown in Fig. 1, the bending movable portion of the insertion guide tubes 2a, 2b and the distal portions of the grasping forceps 51, 52 projecting into the body cavity are located in the front (distal) area of the observation window 21 in the distal portion 6 of the endoscope 1. The insertion guide tubes 2a, 2b and the distal portions of the grasping forceps 51, 52 are displayed in the display area on the screen 40 having a fixed size in various scales according to the value of the field angle W which is currently selected, as shown in Fig. 7A, Fig. 7B, and Fig. 7C.

Therefore, the operator selects the angle of observable field of view by the objective observation optical system 25 according to the type of operation such as an operation, an observation, or a diagnosis as needed, and causes the subject on the monitor screen 40 to be displayed in a scale suitable to the type of operation.

The image shown in Fig. 7A is a state in which the angle of observable field of view W is set to a normal size which is the same as the existing endoscope. In this embodiment, this field angle is "W1" and corresponds, for example, to "130°" shown in Fig. 6. In this case, as shown in Fig. 6, the area including, and wider than, a constant width A which is at a distance of "20 mm" in front of (distal to) the distal end of the endoscope 1 is displayed in the display area of the fixed size on the monitor screen 40.

Therefore, a relatively wide range of the front area of the distal end of the endoscope 1 can be displayed on the monitor screen 40. Such an observation like the existing endoscope is suitable for the case where the operator wants to determine the entire wide range of the front area of the endoscope 1, for example, for the case of insertion of the endoscope or searching of the portion to be treated.

However, when bending the insertion guide tubes 2a, 2b and operating the grasping forceps 51, 52, the grasping forceps 51, 52 are shown in a small scale as shown in Fig. 7A, and hence it is difficult for the operator to operate. In order to view the grasping forceps 51, 52 in a large scale, it is necessary to bend the bending movable portions of the insertion guide tubes 2a, 2b at a large angle in a small radius to bring the same closer to the distal end of the endoscope.

However, since it is necessary to bend the bending portion of the insertion guide tube sharply by a stronger force by the operating wire, the parts at the bending portion or the operation wire is subjected to an excessive force. Also, at the sharply bent portion, the instruments cannot be moved back and forth smoothly. In addition, there are design and/or manufacture limits for the bending portion in terms of having a structure which is capable of being bent sharply.

Therefore, the field angle W is selected so as to be capable of observing in the field angle which enables the operator to view the image in a larger scale, as shown in Fig. 7B. In other words, the field angle is changed into the field angle "W2" which is smaller than the field angle of the existing endoscope. For example, the field angle is assumed to be "90°" shown in Fig. 6. At this time, the entire area of the constant width A at a distance of "40 mm" in front of (distal to) the distal end of the endoscope 1, as shown in Fig. 6, is displayed in the display area of the monitor screen 40 of a fixed size. Therefore, the area apart from the distal end of the endoscope 1 toward the front can be displayed on the monitor screen 40 in an enlarged scale. In the case of the field angle "W2", the area at the position relatively apart from the distal end of the endoscope 1 toward the front can be observed in an enlarged scale. Therefore, since the area apart from the distal end of the endoscope 1 toward the front can be observed in an enlarged scale, the operator can easily operate the grasping forceps 51, 52 by the insertion guide tubes 2a, 2b, so that the images of the grasping forceps 51, 52 are enlarged and can be observed in an viewer-friendly state. The enlarged observation at the field angle of "W2" is particularly suitable for the operation performed by using the therapeutic tools. In other words, the area in front of (distal to) the distal end of the endoscope can be viewed in an enlarged scale without sharply bending the bending portions of the insertion guide tubes 2a, 2b and therefore, avoiding the associated disadvantages of such a sharp bending.

The state of the image shown in Fig. 7C corresponds to a state in which the angle of observable field of view W is set to the narrowest value and simultaneously the distal end of the endoscope is brought to a position, which corresponds to a close-up state. The field angle may be "W2" described above. In other words, the close-up state in which the subject is enlarged to a magnification higher than the existing endoscope can be obtained easily by moving the distal end of the endoscope and selecting the field angle.

As is clear from the fact described above, the size of the image which can be displayed on the monitor screen 40 can be adjusted as needed by the change of the field angle W, in which the angle "W" of the observable field of view by the objective observation optical system 25 is selected.

In particular, when the field angle "W2" is set to obtain the state of the image shown in Fig. 7B, the bending movable portions of the insertion guide tubes 2a, 2b including the first bending portions 17 and the second bending portions 18 can be placed at positions projected apart from the front of the distal portion 6 of the endoscope 1 (distal to the distal end) as shown in solid lines in Fig. 6, whereby an allowance is provided in bending operation of the first bending portions 17 and the second bending portions 18. In addition, the images of the grasping forceps 51, 52 are enlarged, and hence they can be operated while observing in a viewer-friendly state.

Therefore, in this embodiment, the image can be displayed in the screen of an easy-to-observe size, and the tools such as the instrument can be operated easily even without bending the movable portion of the insertion guide tubes 2a, 2b including the first bending portions 17 and the second bending portions 18 strongly and sharply in a small range and a small radius. The parts of the bending portions 17, 18 of the insertion guide tubes 2a, 2b or the operation wire are prevented from being exerted with an excessive force. Furthermore, since the bending portions 17, 18 are not bent sharply, the instruments can be moved back and forth smoothly in the bending portions 17, 18 of the insertion guide tubes 2a, 2b, whereby the operability is further enhanced. In addition, since it is not necessary to provide a structure in which the bending portions 17, 18 can be bent sharply, limit in design or manufacture of the bending portions 17, 18 is advantageously alleviated.

In this embodiment, the entire movement of the manipulator that controls the therapeutic tools during the operation can be secured, and the wide range of observation for viewing the portion near the diseased portion entirely and the observation of the part to be operated in detail in an enlarged scale can be easily selected. In particular, the enlarged observation for determining the movement of the distal end of the manipulator or the therapeutic tool in detail can be easily selected, and hence operation such as moving the endoscope itself toward or away from the subject can be eliminated.

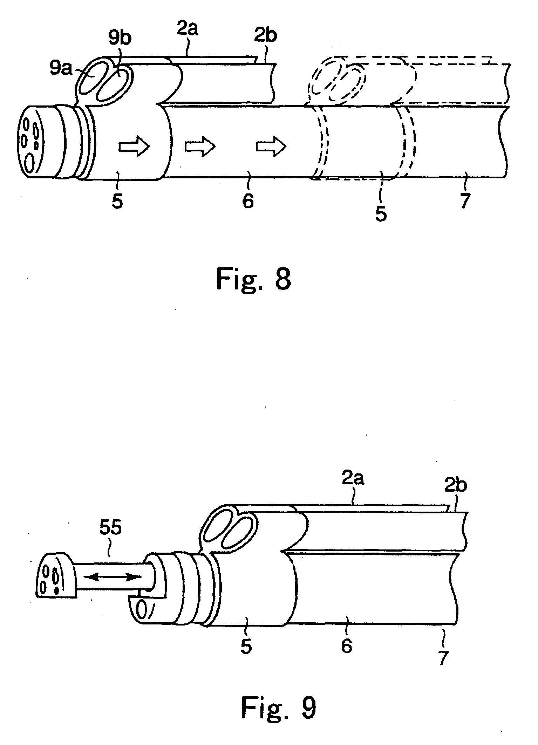

The endoscope apparatus shown in Fig. 8 is of a type in which the fixed position of the connecting member 5 with respect to the insertion portion 4 can be changed. The connecting member 5 can be mounted to a position near the distal end of the distal portion 6 as shown by solid lines in Fig. 8. However, when operating the therapeutic tool by the insertion guide tubes 2a, 2b, the connecting member 5 can be moved backward (proximally) as shown by broken lines in Fig. 8 and mounted to the insertion portion 4 of the endoscope 1. The length of the movable bending areas of the bending portions 17, 18 of the insertion guide tubes 2a, 2b can be increased by the amount corresponding to the amount of retraction of the connecting member 5. Therefore, an allowance is provided in bending of the bending portions 17, 18.

The endoscope shown in Fig. 9 is configured in such a manner that an objective observation optical system unit 55 which is to be assembled into the distal portion 6 of the endoscope 1 can be projected from and retracted into the distal end of the distal portion 6 in the fore-and-aft direction. When the insertion guide tubes 2a, 2b are used, and the insertion guide tubes 2a, 2b are bent, the objective observation optical system unit 55 is projected forward from the distal end of the distal portion 6, whereby the length of the bending movable areas of the bending portions 17, 18 of the insertion guide tubes 2a, 2b can be increased correspondingly. Therefore, the bent portions 17, 18 can be bent with allowance.

In the endoscope of the type shown in Fig. 8 or Fig. 9 described above as well, when the mechanism for changing the field angle of the objective observation optical system 25 as described above is built in, the advantage of the mechanism that change the field angle can be obtained.

In this embodiment, since the focal distance does not change before and after enlargement, there is an advantage in that the image can be viewed in an enlarged scale without moving the endoscope itself, for example, back and forth.

According to this embodiment, since the scale of enlargement can be converted in a state in which the position of the endoscope 1 is fixed, and hence the objective portion can be viewed in an enlarged scale without changing the position of the endoscope 1, or the entire portion can be overviewed, operability is enhanced.

According to this embodiment described above, the objective observation optical system 25 employs a structure of a scope with magnification function in which the single concave lens at the midpoint of the lens group is moved. However, in actual use, the focus position of the objective observation optical system 25 does not have to be the best position, and as long as it is in approximate focus, there is no problem in the actual use and hence is acceptable. It is also possible to employ a type having a zooming mechanism in which the focal distance (the position in focus) is changed according to the change of the angle of observable field of view. Furthermore, it is also possible to design the same so that the image can be enlarged by reducing the field angle and the object at the closer position can be focalized, so that enlargement can be achieved by reducing the field angle.

Although an example in which the electric actuator 36 is employed as the operating mechanism for changing the field angle is shown in this embodiment, other means are possible. For example, it is also possible to employ a mechanical operating system in which an operating element such as an operating knob provided at the control section on the operator's side is operated and the operating force is transmitted to the movable lens retaining frame 28b by the operating force transmitting mechanism such as a wire.

Furthermore, although the insertion guide tubes 2a, 2b are guided by the use of the guide tubes 3a, 3b in this embodiment, it is also possible to mount the insertion guide tubes 2a, 2b directly to the connecting member 5 without using the guide tubes 3a, 3b.

Although the range of the angle of observable field of view taken into the observation optical system of the endoscope 1 may be set to a wide range as described above, when the angle of field of view corresponding to the length of the longest diagonal line in the image pickup area of the image pickup device 26 is set, for example, to the range from 60° to 100° in an enlarged state, practicability and usability are achieved.

Also, when the field angle is set to the range from 100° to 150° for the normal observation (at a wide angle), and to the range from 60° to 100° in an enlarged state, the optimal state of observation can easily be respectively obtained.

In addition, when observing in an enlarged state by the observation optical system, it is preferable that the focal depth before and after the enlargement is in the same focal depth range.

Normally, since the objective portion is located at a distance of about 40 mm, it is preferable that the focal depth is located at a position in the vicinity of the position 40 mm apart from the position of the distal end of the distal-most lens of the observation optical system in terms of actual use of the endoscope.

The focal depth can be represented generally by the distance from the distal end surface of the lens located at the distal-most position of the observation optical system. The focal depth is not the distance of one point, but the range in which the image seems to be substantially in focus, and hence the focal depth includes a certain range which is represented by the position referred to as the focal depth, which moves apart toward the front from the position of the distal end of the distal-most lens in the observation optical system.

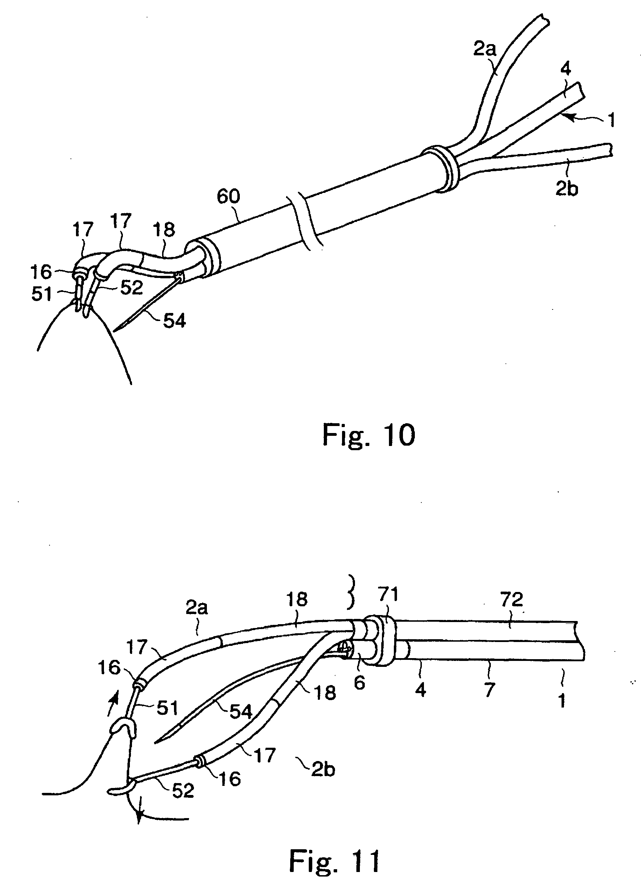

Subsequently, referring now to Fig. 10, an endoscope apparatus according to a second embodiment will be described. This embodiment employs an over-tube 60 as an auxiliary tool for detachably positioning and supporting the guide tubes 2a, 2b and the insertion portion 4 of the endoscope 1. The guide tubes 2a, 2b and the insertion portion 4 of the endoscope 1 are guided by the over-tube 60, and the endoscope 1 and the insertion guide tubes 2a, 2b are positioned and retained in a state of being correlated with each other.

In this embodiment, since the over-tube 60 is employed, it is easy to replace the endoscope 1 and the insertion guide tubes 2a, 2b. Other parts are the same as the first embodiment described above. Therefore, the same effects as described above are obtained in this embodiment as well.

Referring now to Fig. 11, an endoscope treatment apparatus according to a third embodiment will be described. In this embodiment, part of the bending portion 18 near the operator's side is connected to the distal portion 6 of the endoscope 1 using a connecting member 71 while avoiding interference with the bending portions 17, 18 of the insertion guide tubes 2a, 2b. Insertion portions 72 of the insertion guide tubes 2a, 2b are integrated into one piece. The bending portions 17, 18 of the two insertion guide tubes 2a, 2b are respectively extended from the distal end of the integrated insertion portion 72. Then, the endoscope 1 and the insertion guide tubes 2a, 2b are positioned and detachably bundled by the connecting member 71 in a state in which the insertion portions 72 of the insertion guide tubes 2a, 2b are laid along the insertion portion 4 of the endoscope 1.

According to this embodiment, the effects as described above can be obtained, and in addition, a positional relationship between the bending portions 17, 18 of the two insertion guide tubes 2a, 2b can be determined. Therefore, the operation of the bending portions 17, 18 can advantageously be performed easily. Other parts are the same as the first embodiment described above. Therefore, with this embodiment as well, the above-described effects can be obtained.

## Claims

1. An endoscope apparatus comprising:
an endoscope (1) having an observation optical system (25);
wherein, in the observation optical system (21, 25-27, 29, 30, 34), an angle of observable field of view for observing the therapeutic tool can be changed, and the endoscope further has an operating mechanism (36, 37) adapted for changing the angle of observation field of view,
**characterized in that**
the endoscope further comprises an insertion guide tube (2a, 2b) adapted for guiding a therapeutic tool into a body cavity, wherein the insertion guide tube is detachably positioned and supported in a predetermined position relative to the endoscope (1);
and
the focal depth of the observation optical system (21, 25-27, 29, 30, 34) after changing of the angle of observable field of view at least partially overlaps with that of before changing thereof.

2. An endoscope apparatus according to Claim 1, wherein the insertion guide tube (2a, 2b) has a bending function and is positioned and supported with an insertion portion (4) of the endoscope (1).

3. An endoscope apparatus according to Claim 2, wherein the predetermined position comprises an endoscope position located distally to a bending portion (16, 17) of the endoscope (1) corresponding to an insertion guide tube position located proximally to a bendable area of the insertion guide tube (2a, 2b).

4. An endoscope apparatus according to Claim 1, wherein an image pickup device (26) adapted for picking up an image of the observable field of view into the endoscope apparatus is further provided, and wherein the angle of field of view corresponds to a length of the longest diagonal line in an image pickup area of the image pickup device (26) set to a range between 60 DEG and 100 DEG for a narrow angle mode.

5. An endoscope apparatus according to Claim 1, wherein the observation optical system (21, 25-27, 29, 30, 34) further comprises an image pickup device for picking up an image of the observable field of view into the endoscope (1), and the angle of field of view corresponds to a length of a longest diagonal line in an image pickup area of the image pickup device set to a range between 100 DEG and 150 DEG for a wide angle mode, and between 60 DEG and 100 DEG for a narrow angle mode.

6. An endoscope apparatus according to Claim 1, wherein the focal depth of the observation optical system (26) includes a position 40 mm distally from a position of a distal end of a lens (30) at a distal-most position of the observation optical system (26).

7. An endoscope apparatus according to Claim 1 comprising an auxiliary member for detachably positioning and supporting the insertion guide tube (2a, 2b) and the endoscope (1) in the predetermined position.

8. An endoscope apparatus according to Claim 1, wherein the insertion guide tube (2a, 2b) comprises a bending portion (17, 18) adapted to be operable by an actuation by an operator.

9. An endoscope apparatus according to Claim 8, wherein the insertion portion (4) is located proximally to the bending portion (17, 18) of the insertion guide tube (2a, 2b) and is inserted into a flexible over-tube (5) so that the insertion guide tube (2a, 2b) is positioned and supported relative to the endoscope (1).

10. An endoscope apparatus according to Claim 8, wherein at least part of the insertion portion (4) is located proximally to the bending portion (17, 18) of the insertion guide tube (2a, 2b) and has a structure of being integrally connected.

11. An endoscope apparatus according to claim 1, further comprising: a supporting portion (5) adapted for supporting the insertion guide tube (2a, 2b) to the distal end of the insertion portion (4) and for limiting movement of the insertion guide tube (2a, 2b) in a radial direction to a central axis of the insertion portion (4) of the endoscope (1).

12. The endoscope apparatus of claim 11, wherein the endoscope (1) comprises a bending portion (7), and the supporting portion (5) supports the insertion guide tube (2a, 2b) at a position of the insertion portion (4) distal to the bending portion (7) of the insertion portion (4) of the endoscope (1).

13. The endoscope apparatus of claim 11, wherein the insertion guide tube (2a, 2b) comprises a bending portion (17), and the supporting portion (5) is capable of supporting the insertion guide tube (2a, 2b) at a position proximal to the bending portion (17) of the insertion guide tube (2a, 2b).

14. An endoscope apparatus according to claim 1, further comprising
a supporting portion (5) for supporting the insertion guide tube (2a, 2b) for guiding the therapeutic tool (51, 52) into a body cavity to the distal end of the insertion portion (4), and limiting movement of the insertion guide tube (2a, 2b) in the radial direction to the central axis of the insertion portion (4) of the endoscope (1), the supporting portion (5) can be used in combination with the endoscope (1).

15. The endoscope apparatus according to Claim 1, **characterized by** further comprising a supporting portion (5) for supporting the insertion guide tube (2a, 2b) for guiding the therapeutic tool (51, 52) into a body cavity to the distal end of the insertion portion (4), and limiting movement of the insertion guide tube (2a, 2b) in the radial direction to the central axis of the insertion portion (4) of the endoscope (1),
wherein the supporting portion (5) includes a first opening (9c) having a slit (10) for fitting the distal end of the insertion portion (4) of the endoscope (1) and a second opening (9a, 9b) for supporting and guiding the insertion guide tube (2a, 2b).

16. The endoscope apparatus according to Claim 15, **characterized in that** the second opening (9a, 9b) is fixedly connected to a tube (3a, 3b) for guiding the insertion guide tube (2a, 2b).

## Patentansprüche

1. Endoskopvorrichtung mit:
einem Endoskop (1), das ein optisches Beobachtungssystem (25) aufweist;
wobei in dem optischen Beobachtungssystem (21, 25-27, 29, 30, 34) ein Winkel eines beobachtbaren Sichtfelds zum Beobachten des therapeutischen Instruments veränderbar ist, und das Endoskop ferner einen zum Verändern des Winkels des beobachtbaren Sichtfelds ausgebildeten Betätigungsmechanismus (36, 37) aufweist,
**dadurch gekennzeichnet, dass**
das Endoskop ferner ein Einführführungsrohr (2a, 2b) aufweist, das zum Führen eines therapeutischen Instruments in eine Körperhöhle ausgebildet ist, wobei das Einführführungsrohr in einer vorgegebenen Position relativ zum Endoskop (1) abnehmbar positioniert und gelagert ist;
und
die Fokustiefe des optischen Beobachtungssystems (21, 25-27, 29, 30, 34) nach der Veränderung des Winkels des beobachtbaren Sichtfelds zumindest teilweise die vor dessen Veränderung überdeckt.

2. Endoskopvorrichtung nach Anspruch 1, bei der das Einführführungsrohr (2a, 2b) eine Biegefunktion aufweist und mit einem Einführabschnitt (4) des Endoskops (1) positioniert und gelagert ist.

3. Endoskopvorrichtung nach Anspruch 2, bei der die vorgegebene Position eine Endoskopposition aufweist, die distal zu einem Biegeabschnitt (16, 17) des Endoskops (1) gelegen ist und einer Einführführungsrohrposition entspricht, die proximal zu einem biegsamen Bereich des Einführführungsrohrs (2a, 2b) gelegen ist.

4. Endoskopvorrichtung nach Anspruch 1, bei der ferner eine Bildaufnahmevorrichtung (26) vorgesehen ist, die zum Aufnehmen eines Bildes des beobachtbaren Sichtfelds in die Endoskopvorrichtung ausgebildet ist, und bei der der Winkel des Sichtfelds einer Länge der längsten Diagonale in einem Bildaufnahmebereich der Bildaufnahmevorrichtung (26), eingestellt auf einen Bereich von 60 Grad bis 100 Grad für einen Engwinkel-Modus, entspricht.

5. Endoskopvorrichtung nach Anspruch 1, bei der das optische Beobachtungssystem (21, 25-27, 29, 30, 34) ferner eine Bildaufnahmevorrichtung zum Aufnehmen eines Bildes des beobachtbaren Sichtfelds in das Endoskop (1) aufweist, und bei der der Winkel des Sichtfelds einer Länge einer längsten Diagonale in einem Bildaufnahmebereich der Bildaufnahmevorrichtung, eingestellt auf einen Bereich von 100 Grad bis 150 Grad für einen Weitwinkel-Modus und 60 Grad bis 100 Grad für einen Engwinkel-Modus, entspricht.

6. Endoskopvorrichtung nach Anspruch 1, bei der die Fokustiefe des optischen Beobachtungssystems (26) eine Position beinhaltet, die 40 mm distal zu einer Position eines distalen Endes einer Linse (30) in der am weitesten distalen Position des optischen Beobachtungssystems (26) gelegen ist.

7. Endoskopvorrichtung nach Anspruch 1, mit einem Zusatzelement zum abnehmbaren Positionieren und Lagern des Einführführungsrohrs (2a, 2b) und des Endoskops (1) in der vorgegebenen Position.

8. Endoskopvorrichtung nach Anspruch 1, bei der das Einführführungsrohr (2a, 2b) einen Biegeabschnitt (17, 18) aufweist, der durch Betätigung seitens einer Bedienungsperson betätigt zu werden vermag.

9. Endoskopvorrichtung nach Anspruch 8, bei der der Einführabschnitt (4) proximal zu dem Biegeabschnitt (17, 18) des Einführführungsrohrs (2a, 2b) gelegen ist und in einen flexiblen Overtube (5) eingebracht ist, so dass das Einführführungsrohr (2a, 2b) relativ zum Endoskop (1) positioniert und gelagert ist.

10. Endoskopvorrichtung nach Anspruch 8, bei der wenigstens ein Teil des Einführabschnitts (4) proximal zum Biegeabschnitt (17, 18) des Einführführungsrohrs (2a, 2b) gelegen ist und eine einstückig verbundene Striktur aufweist.

11. Endoskopvorrichtung nach Anspruch 1, ferner mit: einem Lagerbereich (5), der dazu ausgebildet ist, das Einführführungsrohr (2a, 2b) relativ zum distalen Ende des Einführabschnitts (4) zu lagern und die Bewegung des Einführführungsrohrs (2a, 2b) in radialer Richtung relativ zu einer Mittelachse des Einführabschnitts (4) zu begrenzen.

12. Endoskopvorrichtung nach Anspruch 11, bei der das Endoskop (1) einen Biegeabschnitt (7) aufweist und der Lagerbereich (5) das Einführführungsrohr (2a, 2b) lagert in einer Position des Einführabschnitts (4) distal zum Biegeabschnitt (7) des Einführabschnitts (4) des Endoskops (1).

13. Endoskopvorrichtung nach Anspruch 11, bei der das Einführführungsrohr (2a, 2b) einen Biegeabschnitt (17) aufweist und der Lagerbereich (5) dazu ausgebildet ist, das Einführführungsrohr (2a, 2b) in einer Position proximal zum Biegeabschnitt (17) des Einführführungsrohrs (2a, 2b) zu lagern.

14. Endoskopvorrichtung nach Anspruch 1, ferner mit
einem Lagerbereich (5) zum Lagern des zum Führen des therapeutischen Instruments (51, 52) in eine Körperhöhle dienenden Einführführungsrohrs (2a, 2b) relativ zum distalen Ende des Einführabschnitts (4) und zum Begrenzen der Bewegung des Einführführungsrohrs (2a, 2b) in radialer Richtung relativ zur Mittelachse des Einführabschnitts (4) des Endoskops (1), wobei der Lagerbereich (5) in Kombination mit dem Endoskop (1) verwendbar ist.

15. Endoskopvorrichtung nach Anspruch 1, **dadurch gekennzeichnet, dass** es ferner einen Lagerbereich (5) aufweist, zum Lagern des zum Führen des therapeutischen Instruments (51, 52) in eine Körperhöhle dienenden Einführführungsrohrs (2a, 2b) relativ zum distalen Ende des Einführabschnitts (4) und zum Begrenzen der Bewegung des Einführführungsrohrs (2a, 2b) in radialer Richtung relativ zur Mittelachse des Einführabschnitts (4) des Endoskops (1),
wobei der Lagerbereich (5) eine erste Öffnung (9c) mit einem Schlitz (10) zum Einpassen des distalen Endes des Einführabschnitts (4) des Endoskops (1) und eine zweite Öffnung (9a, 9b) zum Lagern und Führen des Einführführungsrohrs (2a, 2b) aufweist.

16. Endoskopvorrichtung nach Anspruch 15, **dadurch gekennzeichnet, dass** die zweite Öffnung (9a, 9b) fest mit einem Rohr (3a, 3b) zum Führen des Einführführungsrohrs (2a, 2b) verbunden ist.

## Revendications

1. Appareil d'endoscope comprenant :
un endoscope (1) comportant un système optique d'observation (25) ;
dans lequel, dans le système optique d'observation (21, 25 à 27, 29, 30, 34), un angle de champ de vision observable destiné à observer l'outil thérapeutique peut être changé, et
l'endoscope comprend en outre un mécanisme d'actionnement (36, 37) adapté pour changer l'angle du champ de vision d'observation,
**caractérisé en ce que**
l'endoscope comprend en outre un tube de guidage d'insertion (2a, 2b) adapté pour guider un outil thérapeutique dans une cavité de corps, dans lequel le tube de guidage d'insertion est positionné de manière détachable et supporté dans une position prédéterminée par rapport à l'endoscope (1) ;
et
la profondeur focale du système optique d'observation (21, 25 à 27, 29, 30, 34) après le changement de l'angle du champ de vision observable chevauche au moins partiellement celle avant le changement de celui-ci.

2. Appareil d'endoscope selon la revendication 1, dans lequel le tube de guidage d'insertion (2a, 2b) possède une fonction de cintrage et est positionné et supporté avec une partie d'insertion (4) de l'endoscope (1).

3. Appareil d'endoscope selon la revendication 2, dans lequel la position prédéterminée comprend une position d'endoscope placée de manière distale vis-à-vis d'une partie de cintrage (16, 17) de l'endoscope (1) correspondant à une position du tube de guidage d'insertion placée de manière proximale vis-à-vis d'une zone cintrable du tube de guidage d'insertion (2a, 2b).

4. Appareil d'endoscope selon la revendication 1, dans lequel un dispositif (26) de capture d'image adapté pour capturer une image du champ de vision observable dans l'appareil d'endoscope est en outre prévu, et dans lequel l'angle de champ de vision correspond à une longueur de la ligne diagonale la plus longue dans une zone de capture d'image du dispositif (26) de capture d'image établie à une plage entre 60° et 100° pour un mode angle étroit.

5. Appareil d'endoscope selon la revendication 1, dans lequel le système optique d'observation (21, 25 à 27, 29, 30, 34) comprend en outre un dispositif de capture d'image destiné à capturer une image du champ de vision observable dans l'endoscope (1), et l'angle du champ de vision correspond à une longueur de la ligne diagonale la plus longue dans une zone de capture d'image du dispositif de capture d'image établie à une plage entre 100° et 150° pour un mode grand-angle, et entre 60° et 100° pour un mode angle étroit.

6. Appareil d'endoscope selon la revendication 1, dans lequel la profondeur focale du système optique d'observation (26) comprend une position distale de 40 mm par rapport à une position d'une extrémité distale d'une lentille (30) au niveau de la position la plus distale du système optique d'observation (26).

7. Appareil d'endoscope selon la revendication 1, comprenant un élément auxiliaire destiné à positionner de manière détachable et supporter le tube de guidage d'insertion (2a, 2b) et l'endoscope (1) dans la position prédéterminée.

8. Appareil d'endoscope selon la revendication 1, dans lequel le tube de guidage d'insertion (2a, 2b) comprend une partie de cintrage (17, 18) adaptée pour être actionnable par un actionnement réalisé par un opérateur.

9. Appareil d'endoscope selon la revendication 8, dans lequel la partie d'insertion (4) est placée de manière proximale vis-à-vis de la partie de cintrage (17, 18) du tube de guidage d'insertion (2a, 2b) et est insérée dans une gaine (5) flexible de sorte que le tube de guidage d'insertion (2a, 2b) est positionné et supporté par rapport à l'endoscope (1).

10. Appareil d'endoscope selon la revendication 8, dans lequel au moins une partie de la partie d'insertion (4) est placée de manière proximale vis-à-vis de la partie de cintrage (17, 18) du tube de guidage d'insertion (2a, 2b) et présente une structure consistant à être connectées de manière solidaire.

11. Appareil d'endoscope selon la revendication 1, comprenant : une partie de support (5) adaptée pour supporter le tube de guidage d'insertion (2a, 2b) sur l'extrémité distale de la partie d'insertion (4) et pour limiter le déplacement du tube de guidage d'insertion (2a, 2b) dans une direction radiale vers un axe central de la partie d'insertion (4) de l'endoscope (1).

12. Appareil d'endoscope selon la revendication 11, dans lequel l'endoscope (1) comprend une partie de cintrage (7), et la partie de support (5) supporte le tube de guidage d'insertion (2a, 2b) au niveau d'une position de la partie d'insertion (4) distale vis-à-vis de la partie de cintrage (7) de la partie d'insertion (4) de l'endoscope (1).

13. Appareil d'endoscope selon la revendication 11, dans lequel le tube de guidage d'insertion (2a, 2b) comprend une partie de cintrage (17), et la partie de support (5) est capable de supporter le tube de guidage d'insertion (2a, 2b) au niveau d'une position proximale vis-à-vis de la partie de cintrage (17) du tube de guidage d'insertion (2a, 2b).

14. Appareil d'endoscope selon la revendication 1, comprenant en outre
une partie de support (5) destinée à supporter le tube de guidage d'insertion (2a, 2b) pour guider l'outil thérapeutique (51, 52) dans une cavité de corps vers l'extrémité distale de la partie d'insertion (4), et limiter le déplacement du tube de guidage d'insertion (2a, 2b) dans la direction radiale vers l'axe central de la partie d'insertion (4) de l'endoscope (1), la partie de support (5) pouvant être utilisée en combinaison avec l'endoscope (1).

15. Appareil d'endoscope selon la revendication 1, **caractérisé en ce qu'**il comprend en outre une partie de support (5) destinée à supporter le tube de guidage d'insertion (2a, 2b) pour guider l'outil thérapeutique (51, 52) dans une cavité de corps vers l'extrémité distale de la partie d'insertion (4), et limiter le déplacement du tube de guidage d'insertion (2a, 2b) dans la direction radiale vers l'axe central de la partie d'insertion (4) de l'endoscope (1),
dans lequel la partie de support (5) comprend une première ouverture (9c) comportant une fente (10) destinée à ajuster l'extrémité distale de la partie d'insertion (4) de l'endoscope (1) et une deuxième ouverture (9a, 9b) destinée à supporter et à guider le tube de guidage d'insertion (2a, 2b).

16. Appareil d'endoscope selon la revendication 15, **caractérisé en ce que** la deuxième ouverture (9a, 9b) est raccordée de manière fixe à un tube (3a, 3b) destiné à guider le tube de guidage d'insertion (2a, 2b).
